**Europäisches Patentamt**

**European Patent Office**

**Office européen des brevets**

(19)

(11) Veröffentlichungsnummer: **0 074 024**
**B1**

(12) # EUROPÄISCHE PATENTSCHRIFT

(45) Veröffentlichungstag der Patentschrift:
05.12.84

(51) Int. Cl.³: **C 07 C 143/60**

(21) Anmeldenummer: **82107830.0**

(22) Anmeldetag: **26.08.82**

(54) Verfahren zur Isolierung von 1-Naphthylamin-4,8-disulfonsäure.

(30) Priorität: **07.09.81 DE 3135391**

(43) Veröffentlichungstag der Anmeldung:
**16.03.83 Patentblatt 83/11**

(45) Bekanntmachung des Hinweises auf die Patenterteilung:
**05.12.84 Patentblatt 84/49**

(84) Benannte Vertragsstaaten:
**CH DE GB LI**

(56) Entgegenhaltungen:
**EP - A - 0 009 743**
**EP - A - 0 037 511**
**DE - A - 2 826 842**

(73) Patentinhaber: **BAYER AG, Konzernverwaltung RP**
**Patentabteilung, D-5090 Leverkusen 1 Bayerwerk (DE)**

(72) Erfinder: **Blank, Heinz Ulrich, Dr., Am Geusfelde 35,**
**D-5068 Odenthal (DE)**
Erfinder: **Behre, Horst, Dr., Zur alten Linde 12,**
**D-5068 Odenthal (DE)**
Erfinder: **Linden, Hans Werner, Dr., Heymannstrasse 38,**
**D-5090 Leverkusen 1 (DE)**
Erfinder: **Mentzel, Werner, Dr., Morgengraben 5,**
**D-5000 Köln 80 (DE)**

## Beschreibung

Die Erfindung betrifft ein Verfahren zur Isolierung von 1-Naphthylamin-4,8-disulfonsäure aus Sulfonierungsgemischen, die diese enthalten, durch Eintragen in vorgewärmtes Wasser.

1-Naphthylamin-4,8-disulfonsäure ist ein wichtiges Zwischenprodukt für die Herstellung von Farbstoffen.

Es sind bereits verschiedene Verfahren zur Herstellung von 1-Naphthylamin-4,8-disulfonsäure bekannt. So ist beispielsweise in der FR-PS Nr. 1490508 ein Verfahren beschrieben, gemäss dem man 1-Naphthylamin-8-sulfonsäure in alkoholisch-wässeriger Lösung mit Mangandioxid und Natriumhydrogensulfit sulfoniert. Dieses Verfahren hat jedoch den Nachteil, dass teures Mangandioxid verwendet wird, eine schlechte Raum-Zeit-Ausbeute erzielt wird und grosse Mengen salzhaltiger Mutterlauge anfallen. Zur Aufarbeitung ist weiterhin eine Abtrennung des überschüssigen Mangandioxid und ein vollständiges Verkochen von Schwefeldioxid erforderlich. Dieses Verfahren kommt daher für die Übertragung in den technischen Massstab nicht in Betracht.

Andere Verfahren gehen von Naphthalin aus, welches zu Naphthalin-1,5-disulfonsäure sulfoniert, in 4-Stellung nitriert und schliesslich zu 1-Naphthylamin-4,8-disulfonsäure reduziert wird. Solche Verfahren sind beispielsweise in Beilstein, Hauptwerk, Bd. XIV, S. 787, „Ullmanns Enzyklopädie der technischen Chemie", 3. Aufl., Bd. 12, S. 629 (1960) und in DRP 45 776, referiert in Friedländer, Bd. 2, S. 253, beschrieben. Die Ausbeuten an isolierter Disulfonsäure liegen bei nur knapp über 30% der theoretischen Ausbeute.

Von technischem Interesse sind die Verfahren, bei denen die Sulfonierung von 1-Naphthylamin-8-sulfonsäure mit Oleum vorgenommen wird. Solche Verfahren sind beispielsweise in Beilstein, Hauptwerk, Bd. XIV, S. 787, DRP 40 571, referiert in Friedländer, Bd. 1, S. 394 und in „Office Techn. Services Reports", 74, 197, referiert in „Ullmanns Enzyklopädie der technischen Chemie", 3. Aufl., Bd. 12, S. 629 (1960), beschrieben.

Gemäss den Angaben in DRP 40 571 soll 1-Naphthylamin-8-sulfonsäure durch Eintragen in 10%iges Oleum unter Vermeidung von Erwärmung und anschliessendem Erwärmen auf dem Wasserbad, bis eine Probe vollständig in Wasser löslich ist, zu 1-Naphthylamin-4,8-disulfonsäure sulfoniert werden. Bei diesem Verfahren bilden sich grosse Mengen an isomeren Disulfonsäuren und höher sulfonierten Produkten, die ein aufwendiges Umlösen zur Gewinnung einer reinen 1-Naphthylamin-4,8-disulfonsäure erforderlich machen.

Gemäss dem „Office Techn. Services Reports", 74, 197 wird 1-Naphthylamin-8-sulfonsäure in 100%iger Schwefelsäure als Reaktionsmedium mit 65%igem Oleum in Gegenwart von wasserfreiem Natriumsulfat sulfoniert. Dazu werden in die Schwefelsäure bei 20 bis 30°C umschichtig, in sechs Portionen Natriumsulfat, 1-Naphthylamin-8-sulfonsäure und etwa die Hälfte der insgesamt benötigten Oleummenge eingetragen. Zum Schluss wird das restliche Oleum zugegeben und etwa 40 h bei 20 bis 30°C nachgerührt. Das Molverhältnis $SO_3 : Na_2SO_4 : 1$-Naphthylamin-8-sulfonsäure beträgt hierbei 3,3 : 0,5 : 1. Das erhaltene Sulfonierungsgemisch wird in Wasser gedrückt und die 1-Naphthylamin-4,8-disulfonsäure mit Natriumchlorid ausgesalzen. Das Produkt wird abgesaugt und der Presskuchen wird umgelöst. Die Ausbeute wird mit 82% der theoretischen Ausbeute angegeben.

Die Nacharbeitung dieser Vorschrift ergab jedoch isolierte Ausbeuten von weniger als 60% der theoretischen Ausbeute, auch wenn auf ein Umlösen verzichtet wird. Nachteilig an dem genannten Verfahren sind die schlechte Ausbeute, die zum Aussalzen benötigten grossen Mengen Kochsalz, die unbefriedigende Filtrierbarkeit des Rohproduktes und das erforderliche Umlösen der Rohware, um eine reines und salzfreies Produkt zu erhalten. Bei der Sulfonierung entstehen weiterhin grössere Mengen an Nebenprodukten, wie 1-Naphthylamin-3,8-disulfonsäure, 1-Naphthylamin-6,8-disulfonsäure und 1,8-Naphthsultam-2,4-disulfonsäure, die alle bei der Isolierung teilweise bei der gewünschten 1-Naphthylamin-4,8-disulfonsäure verbleiben.

Es wurde nun ein Verfahren zur Isolierung von 1-Naphthylamin-4,8-disulfonsäure aus diese enthaltenden Sulfonierungsgemischen durch Eintragen in Wasser gefunden, das dadurch gekennzeichnet ist, dass man das die 1-Naphthylamin-4,8-disulfonsäure enthaltende Sulfonierungsgemisch in Gegenwart von mindestens 1 Eq Alkalimetallionen so in gegebenenfalls vorgewärmtes Wasser einträgt, dass bei Beginn der Kristallisation eine Temperatur von mindestens 100°C erreicht wird, und die Trennung von 1-Naphthylamin-4,8-disulfonsäure und Mutterlauge bei einer Temperatur von höchstens 70°C vornimmt.

Das Sulfonierungsgemisch kann in Wasser von Raumtemperatur oder in vorgewärmtes Wasser, bevorzugt in vorgewärmtes Wasser, eingetragen werden.

Als Temperatur des vorgewärmten Wassers sei beispielsweise 40°C bis zum Siedepunkt bei Normaldruck, bevorzugt mindestens 70, besonders bevorzugt mindestens 80°C, genannt.

Das Sulfonierungsgemisch wird so in dieses vorgewärmte Wasser eingetragen, dass bei Beginn der Kristallisation der 1-Naphthylamin-4,8-disulfonsäure eine Temperatur von mindestens 100, beispielsweise 100°C bis wenig unter dem Siedepunkt der entstehenden Suspension, erreicht wird. Eine vorteilhafte Variante des Eintragens des Sulfonierungsgemisches in das vorgewärmte Wasser besteht darin, dass man nur einen Teil dieses Wassers, beispielsweise 20 bis 50% der Gesamtmenge, erwärmt und vorlegt und sodann das Sulfonierungsgemisch und das restliche, nicht vorgewärmte Wasser simultan in den vorgewärmten Teil des Wassers einträgt.

Erfindungsgemäss wird die Gesamtmenge an Wasser, mit der das Sulfonierungsgemisch vereinigt wird, so bemessen, dass die Schwefelsäurekonzentration in der Mutterlauge der entstehenden Suspension etwa 10 bis 70 Gew.-%, bevorzugt 30 bis 60 Gew.-%, beträgt.

Die 1-Naphthylamin-4,8-disulfonsäure wird aus der entstandenen Suspension durch Filtration, Dekantieren oder Zentrifugieren, bevorzugt durch Filtration, abgetrennt. Vor Beginn der Abtrennung, beispielsweise der durch Filtration, wird die Suspension auf eine Temperatur von höchstens 70° C gebracht. In bevorzugter Weise wird die 1-Naphthylamin-4,8-disulfonsäure bei einer Temperatur von 10 bis 30° C abfiltriert.

Das erfindungsgemässe Verfahren ist auf die Isolierung von 1-Naphthylamin-4,8-disulfonsäure aus allen Sulfonierungsgemischen anwendbar, in denen mit Schwefelsäure und Schwefeltrioxid unter verschiedenen sonstigen Reaktionsbedingungen sulfoniert wird, wenn bei der wässerigen Aufarbeitung der Sulfonierungsgemische mindestens 1 Eq Alkalimetallionen anwesend ist. Die 1-Naphthylamin-4,8-disulfonsäure wird im erfindungsgemässen Verfahren als saures Monoalkalisalz gewonnen.

Als Alkalimetallionen seien beispielsweise Lithium-, Natrium-, Kalium-, Rubidium- oder Cäsiumionen sowie die Ammoniumionen genannt. In bevorzugter Weise seien die Natrium-, Kalium- oder Ammoniumionen, besonders bevorzugt die Natriumionen, genannt. Entsprechend wird die 1-Naphthylamin-4,8-disulfonsäure beispielsweise als Mononatrium-, Monokalium- oder Monoammoniumsalz gewonnen.

Diese Alkalimetallionen können beispielsweise in Form der zugehörigen Sulfate oder Hydrogensulfate in der wässerigen Suspension anwesend sein. In bevorzugter Weise wird mit den Sulfaten dieser Alkalimetallionen gearbeitet.

Die Alkalimetallionen liegen in einer Menge von mindestens 1 Eq pro Mol 1-Naphthylamin-4,8-disulfonsäure vor, beispielsweise in einer Menge von 1 bis 10 Eq, bevorzugt 1,1 bis 6 Eq, besonders bevorzugt 1,2 bis 4 Eq. Für das erfindungsgemässe Verfahren ist es ohne Belang, ob die Alkalimetallionen in Form der genannten Salze mit dem Sulfonierungsgemisch in die Suspension gelangen oder unabhängig vom Sulfonierungsgemisch in diese Suspension gegeben werden. Bevorzugt gelangen die Alkalisalze mit dem Sulfonierungsgemisch in die Suspension.

In bevorzugter Weise werden demnach Sulfonierungsgemische im erfindungsgemässen Verfahren eingesetzt, die in Gegenwart von mindestens 1 Eq Alkalimetallionen pro Mol der zu sulfonierenden 1-Naphthylamin-8-sulfonsäure durchgeführt werden. Eine solche Sulfonierung kann beispielsweise so durchgeführt werden, dass man umschichtig ein oder mehrere Alkali und/oder Ammoniumsalze — bevorzugt die Sulfate — 1-Naphthylamin-8-sulfonsäure und gegebenenfalls verdünntes $SO_3$ in vorgelegte Schwefelsäure gibt.

Es wurde nun gefunden, dass man besonders günstige Ergebnisse beim Sulfonierungsumsatz, bei der Selektivität zur gewünschten 1-Naphthylamin-4,8-disulfonsäure und bei der nachfolgenden wässerigen Aufarbeitung erzielt, wenn man

zur Sulfonierung in $H_2SO_4$ als Reaktionsmedium durch das beschriebene umschichtige Einsetzen der Einsatzstoffe insgesamt 1,5 bis 3 mol $SO_3$ und 0,6 bis 1,5 mol Alkalisulfat pro Mol 1-Naphthylamin-8-sulfonsäure einsetzt.

Die Erfindung betrifft daher in bevorzugter Weise ein Verfahren zur Isolierung von 1-Naphthylamin-4,8-disulfonsäure aus diese Säure enthaltenden Sulfonierungsgemischen, das dadurch gekennzeichnet ist, dass man als Sulfonierungsgemische solche verwendet, wie sie bei dem bekannten Sulfonieren in Schwelfäure durch umschichtiges Eintragen von $SO_3$, Alkalisulfat und 1-Naphthylamin-8-sulfonsäure bei Anwendung von insgesamt 1,5 bis 3 mol $SO_3$ und 0,6 bis 1,5 mol Alkalisulfat pro Mol 1-Naphthylamin-8-sulfonsäure erhalten werden und diese ausreagierten Sulfonierungsgemische in der beschriebenen Weise so in gegebenenfalls vorgewärmtes Wasser einträgt, dass bei Beginn der Kristallisation eine Temperatur von mindestens 100° C erreicht wird, und die Trennung von 1-Naphthylamin-4,8-disulfonsäure und Mutterlauge bei einer Temperatur von höchstens 70° C vornimmt.

$SO_3$ kann als solches oder in Form von Lösungen in Schwefelsäure eingesetzt werden. Lösungen von $SO_3$ in Schwefelsäure sind unter der Bezeichnung Oleum bekannt. Oleum kann verschiedene $SO_3$-Gehalte aufweisen. Erfindungsgemäss wird bevorzugt ein Oleum mit 50 bis 80 Gew.-% $SO_3$, bezogen auf das Gesamtgewicht des Oleums, eingesetzt.

Die Gesamtmenge an $SO_3$ beträgt 1,5 bis 3 mol pro Mol 1-Naphthylamin-8-sulfonsäure. Das $SO_3$, gegebenenfalls in der Form von Oleum, kann hierbei vollständig in Form des umschichtigen Einsetzens aller Ausgangsstoffe verwendet werden. Mit Vorteil kann jedoch auch nur etwa die Hälfte des $SO_3$ bzw. Oleums umschichtig mit den anderen Einsatzstoffen eingetragen werden, wobei die andere Hälfte des $SO_3$ bzw. Oleums nach dem vollständigen Eintragen der anderen Einsatzstoffe, beispielsweise 0,5 bis 3 h danach, zudosiert wird. Bevorzugt werden 2,5 bis 3 mol $SO_3$ pro Mol 1-Naphthylamin-8-sulfonsäure eingesetzt.

Erfindungsgemäss werden weiterhin 0,6 bis 1,5 bevorzugt 0,75 bis 1,0 mol eines oder mehrerer Alkali- und/oder Ammoniumsulfate pro Mol 1-Naphthylamin-8-sulfonsäure eingesetzt. Das Alkali- und/oder Ammoniumsulfat kann gemeinsam mit den anderen Ausgangsstoffen umschichtig, wie oben beschrieben, eingesetzt werden. Es ist jedoch ebenso gut möglich, die Gesamtmenge des Alkali- und/oder Ammoniumsulfats im Reaktionsmedium vorzulegen und erst dann die übrigen Ausgangsstoffe des Verfahrens umschichtig einzusetzen.

Die Sulfonierung der 1-Naphthylamin-8-sulfonsäure wird in wasserfreier Schwefelsäure als Reaktionsmedium vorgenommen. Die Menge der Schwefelsäure beträgt 5 bis 7 mol $H_2SO_4$ pro Mol 1-Naphthylamin-8-sulfonsäure. Wird zum Lösen bzw. Suspendieren eine wasserhaltige Schwefelsäure verwendet und/oder keine trockene, sondern feuchte 1-Naphthylamin-8-sulfonsäure eingesetzt, ist für die Sulfonierung über die oben genannte Menge von 1,5 bis 3 mol Schwefeltrioxid je Mol 1-Naphthylamin-8-sulfonsäure hinaus soviel Schwefeltrioxid, gegebenenfalls in Form von Oleum, zuzusetzen, wie zum Binden des durch die Reagenzien eingebrachten Wassers unter Bildung von Schwefelsäure erforderlich ist.

Das umschichtige Eintragen der Ausgangsstoffe und die Fortführung der Sulfonierung wird beispielsweise bei einer Temperatur von 10 bis 40° C, bevorzugt bei 25 bis 35° C, vorgenommen. Nach beendeter Zugabe aller Ausgangsstoffe für das Verfahren, gegebenenfalls auch nach nachträglicher Zugabe eines Teils des Oleums, lässt man das Reaktionsgemisch bei der angegebenen Temperatur unter Rühren ausreagieren. Die Nachreaktionszeit liegt bei etwa 16 bis etwa 40 h, bevorzugt bei 20 bis 36 h.

Die erfindungsgemäss anfallenden, die 1-Naphthylamin-4,8-disulfonsäure enthaltenden Suspensionen zeichnen sich durch gute Filtrierbarkeit aus. Die erfindungsgemäss hergestellte 1-Naphthylamin-4,8-disulfonsäure hat eine hohe Reinheit von mindestens 97%, bevorzugt von mindestens 98,5%.

*Beispiel 1*

In einem mit Rührer, Tropftrichter, Innenthermometer und Trockenrohr versehenen Reaktionsgefäss werden 630 g 100%ige Schwefelsäure vorgelegt. Dann werden bei 20 bis 30° C unter Kühlen viermal in dieser Reihenfolge jeweils 26,7 g (0,1875 mol) wasserfreies Natriumsulfat, 57 g (0,25 mol) 1-Naphthylamin-8-sulfonsäure 97,8%ig und 46,2 g Oleum 65%ig (=0,375 mol $SO_3$) eingetragen. Das Eintragen jeder Portion aus Natriumsulfat, 1-Naphthylamin-8-sulfonsäure und Oleum soll in 15 bis 20 min erfolgen. Anschliessend wird 1 h bei 30° C gerührt und dann noch 123,1 g Oleum 65%ig (=1,0 mol $SO_3$) in 30 min bei 30° C nachgesetzt; zu Anfang muss noch gekühlt werden, nachher ist schwaches Erwärmen nötig. Danach wird 36 h bei 30° C nachgerührt.

Das fertige Sulfonierungsgemisch wird in eine Vorlage von 1360 g Wasser von 80° C in ca. 1 h eingetragen. Dabei steigt die Temperatur auf ca. 105° C. Nachdem ca. ⅓ des Sulfonierungsgemisches eingetragen sind, beginnt die 1-Naphthylamin-4,8-disulfonsäure auszukristallisieren. Die Temperatur beträgt zu diesem Zeitpunkt 105° C. Nach dem vollständigen Eintragen des Sulfonierungsgemisches wird unter Rühren in ca. 6 h von 105° C auf 20 bis 25° C abgekühlt. Die Suspension wird filtriert und der Filterkuchen 2mal mit je 100 ml Eiswasser schwefelsäurefrei gewaschen und trocken gesaugt.

Es werden 274,3 g Produkt erhalten. Die Hochdruckflüssigkeitschromatographie des isolierten Produktes ergab folgende Zusammensetzung

| | |
|---|---|
| 1-Naphthylamin-8-sulfonsäure | 0,2 Gew.-% |
| 1-Naphthylamin-3,8-disulfonsäure | 0,5 Gew.-% |
| 1-Naphthylamin-4,8-disulfonsäure | 90,4 Gew.-% |
| 1-Naphthylamin-6,8-disulfonsäure | 0,1 Gew.-% |
| 1,8-Naphthsultam-2,4-disulfonsäure | Spur |
| Rest bis 100% | Wasser |

Die Ausbeute an reiner 1-Naphthylamin-4,8-disulfonsäure (Mol-Gew. 303) beträgt 248 g (=81,8% der theoretischen Ausbeute, bezogen auf eingesetzte 1-Naphthylamin-8-sulfonsäure). Die Disulfonsäure liegt als saures Mononatriumsalz vor.

*Beispiel 2*

In einer in Beispiel 1 beschriebenen Apparatur werden 1 260 g 100%ige Schwefelsäure vorgelegt. Dann werden bei 20 bis 30° C unter Kühlen viermal in dieser Reihenfolge jeweils 53,3 g (0,375 mol) wasserfreies Natriumsulfat, 114,1 g (0,5 mol) 1-Naphthylamin-8-sulfonsäure, 97,8%ig, und 92,3 g Oleum 65%ig (=0,75 mol $SO_3$) eingetragen. Das Eintragen jeder Portion aus Natriumsulfat, 1-Naphthylamin-8-sulfonsäure und Oleum soll in 15 bis 20 min erfolgen. Anschliessend wird 1 h bei 30° C gerührt und dann noch 369,3 g Oleum 65%ig (=3,0 mol $SO_3$) in 30 min bei 30° C zugetropft. Danach wird 24 h bei 30° C nachgerührt; zu Anfang muss noch gekühlt werden, nachher ist schwaches Erwärmen nötig.

In einer Rührapparatur werden 500 g Wasser zum Sieden erhitzt, und unter Rühren gibt man simultan 2400 g Wasser von Raumtemperatur und das obige Sulfonierungsgemisch in ca. 1 h zu. Die verdünnte wässerige Lösung bzw. Suspension des Sulfonierungsgemisches wird während des Eintragens durch Erwärmen auf einer Temperatur von 103 bis 105° C gehalten. Nach dem vollständigen Eintragen des Sulfonierungsgemisches wird unter Rühren in ca. 6 h von 103 bis 105° C auf 20 bis 25° C abgekühlt. Die Suspension wird filtriert und der Filterkuchen 2mal mit je 200 ml Eiswasser schwefelsäurefrei gewaschen und trocken gesaugt.

Es werden 545 g lufttrockenes Produkt erhalten.

Die Hochdruckflüssigkeitschromatographie des isolierten Produktes ergab folgende Zusammensetzung

| | |
|---|---|
| 1-Naphthylamin-8-sulfonsäure | 0,3 Gew.-% |
| 1-Naphthylamin-3,8-disulfonsäure | 0,4 Gew.-% |
| 1-Naphthylamin-4,8-disulfonsäure | 86,1 Gew.-% |
| 1-Naphthylamin-6,8-disulfonsäure | 0,3 Gew.-% |
| 1,8-Naphthsultam-2,4-disulfonsäure | Spur |
| Rest bis 100% | Wasser |

Die Ausbeute an reiner 1-Naphthylamin-4,8-disulfonsäure (Mol-Gew. 303) beträgt 469,2 g (=77,4% der theoretischen Ausbeute, bezogen auf eingesetzte 1-Naphthylamin-8-sulfonsäure). Die Disulfonsäure liegt als saures Mononatriumsalz vor.

*Beispiele 3 bis 9*

Es wird, wie in Beispiel 1 beschrieben, sulfoniert, nur werden andere Molverhältnisse Schwefeltrioxid : Natriumsulfat : 1-Naphthylamin-8-sulfonsäure, andere Reaktionstemperaturen und Nachrührzeiten angewendet.

In der nachstehenden Tabelle I sind die bei diesen Sulfonierungsverfahren erhaltenen Ergebnisse zusammengestellt. Die Zusammensetzung der Sulfonierungsgemische wurde mittels Hochdruckflüssigkeitschromatographie bestimmt.

*Tabelle I*

| Beispiel Nr. | Sulfonierungsbedingungen | | | Zusammensetzung des Sulfonierungsgemisches (Mol.-%) | | | | | |
|---|---|---|---|---|---|---|---|---|---|
| | Molverhältnis SO$_3$:Na$_2$SO$_4$:1-NH$_2$-8-S | Reaktions-temperatur (°C) | Nachrühr-zeit (h) | 1-NH$_2$-8 | 1-NH$_2$-3,8 | 1-NH$_2$-4,8 | 1-NH$_2$-6,8 | 1-Naphth-sultam-2,4 | Σ |
| 3 | 3,0:0,54:1 | 20 | 36 | 0,0 | 3,6 | 75,5 | 10,0 | 2,3 | 92,2 |
| 4 | 3,0:0,75:1 | 30 | 24 | <0,5 | 2,7 | 81,0 | 6,9 | 4,6 | 95,7 |
| 5 | 3,0:1,0:1 | 35 | 32 | 1,5 | 2,4 | 75,3 | 6,1 | 8,9 | 94,2 |
| 6 | 2,5:0,54:1 | 30 | 36 | 1,0 | 2,7 | 78,3 | 7,1 | 2,3 | 91,9 |
| 7 | 2,5:0,75:1 | 30 | 36 | 0,6 | 2,3 | 84,6 | 6,1 | 5,4 | 98,0 |
| 8 | 2,5:0,75:1 | 35 | 32 | <0,5 | 2,1 | 80,9 | 5,5 | 8,1 | 96,6 |
| 9 | 2,0:0,5:1 | 40 | 36 | <0,5 | 2,1 | 73,8 | 5,3 | 12,3 | 93,5 |

*Beispiele 10 bis 18*

Es wurde, wie in Beispiel 2 beschrieben, sulfoniert, jedoch wurde nur ein halb so grosser Ansatz durchgeführt. Zur Aufarbeitung wird das gesamte Wasser vorgelegt und auf 80° C erwärmt. Unter Rühren wird das Sulfonierungsgemisch eingetragen, wobei die Temperatur bis auf 105° C ansteigt. Bis zum vollständigen Eintrag des Sulfonierungsgemisches wird die Temperatur der verdünnten wässerigen Lösung bzw. Suspension des Reaktionsgemisches bei ca. 105° C gehalten. Dann wurde unter Rühren gekühlt.

Wie aus der folgenden Tabelle II ersichtlich, wurden bei der Aufarbeitung verschiedene Mengen Wasser vorgelegt und auf diese Weise verschiedene Schwefelsäurekonzentrationen in den wässerigen Lösungen bzw. Suspensionen der Sulfonierungsgemische eingestellt. Ausserdem wird das auskristallisierte 1-Naphthylamin-4,8-disulfonsäuremono-Na-salz bei verschiedenen Temperaturen abgesaugt und anschliessend zweimal mit je 100 ml Eiswasser schwefelsäurefrei gewaschen.

Die Zusammensetzung der unter verschiedenen Bedingungen isolierten 1-Naphthylamin-4,8-disulfonsäuremono-Na-salze wurde mittels Hochdruckflüssigkeitschromatographie ermittelt.

*(Tabelle auf der nächsten Seite)*

*Beispiel 19*

In einer in Beispiel 1 beschriebenen Apparatur werden 1260 g 100%ige Schwefelsäure und 198,2 g (1,5 mol) wasserfreies Ammoniumsulfat vorgelegt. Dann werden bei 20 bis 30° C unter Kühlen viermal in dieser Reihenfolge jeweils 114,1 g (0,5 mol) 1-Naphthylamin-8-sulfonsäure 97,8%ig und 92,3 g Oleum 65%ig (=0,75 mol SO$_3$) eingetragen. Das Eintragen jeder Portion 1-Naphthylamin-8-sulfonsäure und Oleum soll in ca. 15 min erfolgen. Anschliessend wird 1 h bei 30° C gerührt und dann noch 369,3 g Oleum 65%ig (=3,0 mol SO$_3$) in 30 min bei 30° C zugetropft. Danach wird 24 h bei 30° C nachgerührt.

In einer Rührapparatur werden 2900 g Wasser von 80° C vorgelegt und unter Rühren das Sulfonierungsgemisch in ca. 1 h eingetragen. Durch Erwärmen wird die Temperatur der verdünnten wässerigen Lösung bzw. Suspension des Sulfonierungsgemisches auf ca. 105° C gebracht und gehalten. Anschliessend wird im Verlauf von ca. 6 h von ca. 105° C auf 20 bis 25° C abgekühlt. Die Suspension wird filtriert und der Filterkuchen 2mal mit je 150 ml Eiswasser schwefelsäurefrei gewaschen und trocken gesaugt.

Es werden 501 g lufttrockenes Produkt erhalten.

Die Hochdruckflüssigkeitschromatographie des isolierten Produktes ergab folgende Zusammensetzung

| | |
|---|---|
| 1-Naphthylamin-8-sulfonsäure | 0,1 Gew.-% |
| 1-Naphthylamin-3,8-disulfonsäure | 0,5 Gew.-% |
| 1-Naphthylamin-4,8-disulfonsäure | 87,5 Gew.-% |
| 1-Naphthylamin-6,8-disulfonsäure | 0,1 Gew.-% |
| 1,8-Naphthsultam-2,4-disulfonsäure | Spur |
| Rest bis 100% | Wasser |

*Tabelle II*

| Beispiel Nr. | Aufarbeitung | | | | Zusammensetzung des Festoffes (Gew.-%)[1] | | | | Ausbeute 1-Naphthylamin-4,8-disulfonsäure (% d.Th.) |
|---|---|---|---|---|---|---|---|---|---|
| | Mengen vorgelegtem Wasser (g) | H₂SO₄ in der Mutterlauge (Gew.-%) | Absaugtemperatur (°C) | Auswaage (g) | 1-Naphthylamin-8-sulfonsäure | 1-Naphthylamin-3,8-disulfonsäure | 1-Naphthylamin-4,8-disulfonsäure | 1-Naphthylamin-6,8-disulfonsäure | |
| 10 | 1450 | 40 | 20 | 267,0 | 0,2 | 0,5 | 88,0 | 0,3 | 77,5 |
| 11 | 1450 | 40 | 40 | 259,7 | <0,1 | 0,4 | 87,8 | 0,3 | 75,1 |
| 12 | 1450 | 40 | 60 | 260,0 | <0,1 | 0,4 | 87,2 | 0,3 | 74,8 |
| 13 | 967 | 50 | 20 | 249,5 | 0,2 | 0,6 | 92,0 | 0,2 | 75,7 |
| 14 | 967 | 50 | 40 | 262,9 | 0,2 | 0,6 | 82,6 | 0,2 | 71,6 |
| 15 | 967 | 50 | 60 | 250,5 | 0,2 | 0,6 | 85,3 | 0,2 | 70,5 |
| 16 | 645 | 60 | 20 | 267,0 | 0,2 | 0,8 | 85,1 | 0,3 | 74,9 |
| 17 | 645 | 60 | 40 | 257,7 | 0,2 | 0,7 | 84,7 | 0,3 | 72,0 |
| 18 | 645 | 60 | 60 | 239,2 | 0,2 | 0,7 | 82,6 | 0,4 | 65,1 |

[1] Die 1,8-Naphthsultam-2,4-disulfonsäure ist nur in Spuren im kristallinen Produkt enthalten.

Die Ausbeute an reiner 1-Naphthylamin-4,8-disulfonsäure (Mol-Gew. 303) beträgt 438,4 g (=72,3% der theoretischen Ausbeute, bezogen auf eingesetzte 1-Naphthylamin-8-sulfonsäure). Die Disulfonsäure liegt als saures Monoammoniumsalz vor.

*Beispiel 20*

*(Sulfonierung gemäss „Office Techn. Reports", 74, 197)*

In einer in Beispiel 1 beschriebenen Apparatur werden 510 g 100%ige Schwefelsäure vorgelegt. Dann werden bei 20 bis 30° C hintereinander 17,7 g wasserfreies Natriumsulfat, 57,1 g 1-Naphthylamin-8-sulfonsäure 97,8%ig und 31,7 g Oleum 65%ig zugegeben. Die gleichen Mengen werden in der angegebenen Reihenfolge nochmals eingetragen. Dann werden viermal je 8,9 g wasserfreies Natriumsulfat, 28,5 g 1-Naphthylamin-8-sulfonsäure 97,8%ig und 31,7 g Oleum 65%ig eingetragen. Dann sind insgesamt 71 g (0,5 mol) wasserfreies Natriumsulfat, 228,2 g (1 mol) 1-Naphthylamin-8-sulfonsäure 97,8%ig und 190,2 g Oleum 65%ig (=1,55 mol SO₃) eingetragen. Zum Schluss werden noch 215,4 g Oleum 65%ig (=1,75 mol SO₃) nachgegeben. Das Sulfonierungsgemisch wird bei 20 bis 30° C ca. 40 h nachgerührt.

Die durch Hochdruckflüssigkeitschromatographie des fertigen Sulfonierungsproduktes bestimmte Ausbeute an 1-Naphthylamin-4,8-disulfonsäure beträgt 64,4% der theoretischen Ausbeute, bezogen auf eingesetzte 1-Naphthylamin-8-sulfonsäure.

Die Hochdruckflüssigkeitschromatographie des Sulfonierungsgemisches ergab folgende Zusammenstzung

1-Naphthylamin-8-sulfonsäure:
0,15 Gew.-% (=0,8 Mol-%)
1-Naphthylamin-3,8-disulfonsäure:
1,17 Gew.-% (=4,7 Mol-%)
1-Naphthylamin-4,8-disulfonsäure:
16,07 Gew.-% (=64,4 Mol-%)
1-Naphthylamin-6,8-disulfonsäure:
2,97 Gew.-% (=11,9 Mol-%)
1,8-Naphthsultam-2,4-disulfonsäure:
0,36 Gew.-% (=1,2 Mol-%)
Rest bis 100%
Schwefelsäure, Schwefeltrioxid, Natriumsulfat, unbekannte Verbindungen

Das so hergestellte Sulfonierungsgemisch wird auf 2110 g Wasser gegeben, wobei sich die Lösung auf ca. 90° C erwärmt. Die 1-Naphthylamin-4,8-disulfonsäure wird mit 169 g Natriumchlorid ausgesalzen. Nach dem Abkühlen wird filtriert und die rohe Säure mit wenig Eiswasser schwefelsäurefrei gewaschen. Im Vakuumtrockenschrank wird das Produkt 24 h bei 70° C getrocknet. Es werden 316 g Produkt erhalten.

Die Hochdruckflüssigkeitschromatographie des isolierten Produktes ergab folgende Zusammensetzung

1-Naphthylamin-8-sulfonsäure    0,10 Gew.-%

1-Naphthylamin-3,8-disulfonsäure 1,29 Gew.-%
1-Naphthylamin-4,8-disulfonsäure 56,40 Gew.-%
1-Naphthylamin-6,8-disulfonsäure 1,54 Gew.-%
1,8-Naphthsultam-2,4-disulfon-

säure      0,19 Gew.-%
Rest bis 100%      Natriumchlorid und
     -sulfat, Wasser

Die Ausbeute an reiner 1-Naphthylamin-4,8-disulfonsäure (Mol-Gew. 303) beträgt 178,2 g (=58,8% der theoretischen Aubeute, bezogen auf eingesetzte 1-Naphthylamin-8-sulfonsäure). Die Disulfonsäure liegt als saures Mononatriumsalz vor.

## Patentansprüche

1. Verfahren zur Isolierung von 1-Naphthylamin-4,8-disulfonsäure aus diese enthaltenden Sulfonierungsgemischen durch Eintragen in Wasser, dadurch gekennzeichnet, dass man das die 1-Naphthylamin-4,8-disulfonsäure enthaltende Sulfonierungsgemisch in Gegenwart von mindestens 1 Eq Alkalimetallionen so in gegebenenfalls vorgewärmtes Wasser einträgt, dass bei Beginn der Kristallisation eine Temperatur von mindestens 100° C erreicht wird, und die Trennung von 1-Naphthylamin-4,8-disulfonsäure und Mutterlauge bei einer Temperatur von höchstens 70° C vornimmt.

2. Verfahren nach Anspruch 1, dadurch gekennzeichnet, dass man das Sulfonierungsgemisch in mindestens 40° C warmes Wasser einträgt.

3. Verfahren nach den Ansprüchen 1 und 2, dadurch gekennzeichnet, dass man das Wasser nur teilweise als vorgewärmtes Wasser vorlegt und das restliche Wasser simultan mit dem Sulfonierungsgemisch einträgt.

4. Verfahren nach den Ansprüchen 1 bis 3, dadurch gekennzeichnet, dass nach dem Eintragen des Sulfonierungsgemisches in das vorgelegte Wasser in der Mutterlauge der entstehenden Suspension eine Schwefelsäurekonzentration von 10 bis 70 Gew.-% vorliegt.

5. Verfahren nach den Ansprüchen 1 bis 4, dadurch gekennzeichnet, dass in der Mutterlauge der entstehenden Suspension eine Schwefelsäurekonzentration von 30 bis 60 Gew.-% vorliegt.

6. Verfahren nach den Ansprüchen 1 bis 5, dadurch gekennzeichnet, dass man die Trennung von 1-Naphthylamin-4,8-disulfonsäure und Mutterlauge durch Filtration bei 10 bis 30° C vornimmt.

7. Verfahren nach den Ansprüchen 1 bis 6, dadurch gekennzeichnet, dass man als Sulfonierungsgemische solche verwendet, wie sie bei dem bekannten Sulfonieren in Schwefelsäure durch umschichtiges Eintragen von $SO_3$, Alkalisulfat und 1-Naphthylamin-8-sulfonsäure bei Anwendung von insgesamt 1,5 bis 3 mol $SO_3$ und 0,6 bis 1,5 mol Alkalisulfat pro Mol 1-Naphthylamin-8-sulfonsäure erhalten werden, und diese ausreagierten Sulfonierungsgemische so in gegebenenfalls vorgewärmtes Wasser einträgt, dass bei Beginn der Kristallisation eine Temperatur von mindestens 100° C erreicht wird, und die Trennung von 1-Naphthylamin-4,8-disulfonsäure und Mutterlauge bei einer Temperatur von höchstens 70° C vornimmt.

8. Verfahren nach Anspruch 7, dadurch gekennzeichnet, dass man zur Herstellung der zu verwendenden Sulfonierungsgemische 2,5 bis 3 mol $SO_3$ und 0,75 bis 1 mol Alkalisulfat pro Mol 1-Naphthylamin-8-sulfonsäure einsetzt.

9. Verfahren nach den Ansprüchen 7 und 8, dadurch gekennzeichnet, dass man das Sulfonierungsgemisch bei der Zugabe des $SO_3$ und bei der Weiterreaktion bei 10 bis 40° C hält.

10. Verfahren nach den Ansprüchen 7 bis 9, dadurch gekennzeichnet, dass man als Reaktionsmedium für die Sulfonierung die Schwefelsäure in einer solchen Menge anwendet, dass auf 1-Naphthylamin-8-sulfonsäure 5 bis 7 mol $H_2SO_4$ entfallen.

## Claims

1. Process for the isolation of 1-naphthylamine-4,8-disulphonic acid from sulphonation mixtures containing it by introduction into water, characterised in that the sulphonation mixture containing 1-naphthylamine-4,8-disulphonic acid is introduced, in the presence of at least 1 Eq of alkali metal ions, into water, if appropriate preheated, so that a temperature of at least 100° C is reached at the start of crystallisation, and the separation of 1-naphthylamine-4,8-disulphonic acid from the mother liquor is carried out at a temperature not higher than 70° C.

2. Process according to Claim 1, characterised in that the sulphonation mixture is introduced into water heated to at least 40° C.

3. Process according to Claims 1 and 2, characterised in that the water is initially introduced only partly as preheated water, and the remaining water is introduced at the same time as the sulphonation mixture.

4. Process according to Claims 1 to 3, characterised in that the concentration of sulphuric acid in the mother liquor of the suspension being produced after the introduction of the sulphonation mixture into the water initially introduced is 10 to 70% by weight.

5. Process according to Claims 1 to 4, characterised in that the concentration of sulphuric acid in the mother liquor of the suspension being produced is 30 to 60% by weight.

6. Process according to Claims 1 to 5, characterised in that the separation of 1-naphthylamine-4,8-disulphonic acid from the mother liquor is carried out by filtration at 10 to 30° C.

7. Process according to Claims 1 to 6, characterised in that the sulphonation mixtures used are those which are obtained by the known sulphonation in sulphuric acid by introducing in turn $SO_3$, alkali metal sulphate and 1-naphthylamine-8-sulphonic acid while using a total of 1.5 to 3 mol of $SO_3$ and 0.6 to 1.5 mol of alkali metal sulphate per mol of 1-naphthylamine-8-sulphonic acid, and these completely reacted sulphonation mixtures are introduced into water, which has if necessary been preheated, so that a temperature of at least 100° C is reached at the start of crystallisation, and the separation of 1-naphthylamine-4,8-disulphonic acid from the mother liquor is carried out at a temperature not higher than 70° C.

8. Process according to Claim 7, characterised in that for the preparation of the sulphonation mixtures to be used 2.5 to 3 mol of $SO_3$ and 0.75 to 1 mol of alkali metal sulphate are employed per mol of 1-naphthylamine-8-sulphonic acid.

9. Process according to Claims 7 and 8, characterised in that the sulphonation mixture is maintained at 10 to 40° C during the addition of $SO_3$ and during the subsequent reaction.

10. Process according to Claims 7 to 9, characterised in that, as the reaction medium for the sulphonation, the sulphuric acid is used in such an amount that 5 to 7 mol of $H_2SO_4$ are present per 1-naphthylamine-8-sulphonic acid.

## Revendications

1. Procédé pour l'isolement de l'acide 1-naphtylamine-4,8-disulfonique à partir de mélanges de sulfonation le contenant par introduction dans l'eau, caractérisé en ce que l'on introduit le mélange de sulfonation contenant l'acide 1-naphtylamine-4,8-disulfonique dans l'eau éventuellement préchauffée, en présence d'au moins 1 Eq d'ions de métal alcalin, de telle sorte que l'on atteigne au début de la cristallisation une température d'au moins 100° C, et l'on effectue la séparation de l'acide 1-naphtylamine-4,8-disulfonique et de la liqueur mère à une température de 70° C au plus.

2. Procédé selon la revendication 1, caractérisé en ce que l'on introduit le mélange de sulfonation dans de l'eau chaude à au moins 40° C.

3. Procédé selon les revendications 1 et 2, caractérisé en ce que l'on charge l'eau en partie seulement sous forme d'eau préchauffée, et l'on introduit le reste de l'eau simultanément avec le mélange de sulfonation.

4. Procédé selon les revendications 1 à 3, caractérisé en ce que la concentration d'acide sulfurique est de 10 à 70% en poids dans la liqueur mère de la suspension qui se forme après l'introduction du mélange de sulfonation dans l'eau préalablement chargée.

5. Procédé selon les revendications 1 à 4, caractérisé en ce que la concentration en acide sulfurique est de 30 à 60% en poids dans la liqueur mère de la suspension qui se forme.

6. Procédé selon les revendications 1 à 5, caractérisé en ce que l'on effectue la séparation de l'acide 1-naphtylamine-4,8-disulfonique et de la liqueur mère par filtration à 10-30° C.

7. Procédé selon les revendications 1 à 6, caractérisé en ce que l'on utilise comme mélanges de sulfonation ceux qui sont obtenus dans la sulfonation connue dans l'acide sulfurique par introduction, à tour de rôle, de $SO_3$, de sulfate alcalin et d'acide 1-naphtylamine-8-sulfonique, avec application d'un total de 1,5 à 3 mol de $SO_3$ et de 0,6 à 1,5 mol de sulfate alcalin par mole d'acide 1-naphtylamine-8-sulfonique, et on introduit dans l'eau éventuellement préchauffée ce mélange de sulfonation ayant cessé de réagir, de telle sorte que l'on atteigne au début de la cristallisation une température d'au moins 100° C, et l'on effectue la séparation de l'acide 1-naphtyl-amine-4,8-disulfonique et de la liqueur mère à une température de 70° C au plus.

8. Procédé selon la revendication 7, caractérisé en ce que, pour la fabrication des mélanges de sulfonation à utiliser, on utilise 2,5 à 3 mol de $SO_3$ et 0,75 à 1 mol de sulfate alcalin par mole d'acide 1-naphtylamine-8-sulfonique.

9. Procédé selon les revendications 7 et 8, caractérisé en ce que l'on maintient le mélange de sulfonation à 10-40° C pendant l'addition de $SO_3$ et pendant la réaction ultérieure.

10. Procédé selon les revendications 7 à 9, caractérisé en ce que l'on utilise comme milieu de réaction pour la sulfonation l'acide sulfurique en quantité correspondant à 5 à 7 mol de $H_2SO_4$ par rapport à l'acide 1-naphtylamine-8-sulfonique.